Europäisches Patentamt

(19) European Patent Office

Office européen des brevets

(11) Publication number: **0 004 137**
**B1**

(12) # EUROPEAN PATENT SPECIFICATION

(45) Date of publication of patent specification: **11.11.81**

(51) Int. Cl.³: **A 61 K 39/10**

(21) Application number: **79300235.3**

(22) Date of filing: **15.02.79**

(54) Immunogenic cell envelope preparations.

(30) Priority: **17.02.78 GB 646278**

(43) Date of publication of application:
**19.09.79 Bulletin 79/19**

(45) Publication of the grant of the European patent:
**11.11.81 Bulletin 81/45**

(84) Designated Contracting States:
**CH DE FR NL SE**

(56) References cited:
**GB - A - 1 108 906**

(73) Proprietor: **NATIONAL RESEARCH DEVELOPMENT CORPORATION**
**P.O. Box 236 Kingsgate House 66-74 Victoria Street**
**London SW1E 6SL (GB)**

(72) Inventor: **Ellwood, Derek Clifford**
**Upper Close Winterbourne Stoke**
**Salisbury Wiltshire (GB)**
Inventor: **Manchee, Richard Johnathan**
**35, Bulbridge Road Wilton**
**Salisbury Wiltshire (GB)**
Inventor: **Robinson, Andrew**
**42, Broadfield Road East Gomeldon**
**Salisbury Wiltshire (GB)**

(74) Representative: **Bowdery, Anthony Oliver et al,**
**Procurement Executive, Ministry of Defence Patents 1 A (4),Room 1932, 19th Floor Empress State Building Lillie Road**
**London SW6 1TR (GB)**

Courier Press, Leamington Spa, England.

The invention relates to the production of cell envelope preparations having immunogenic properties and potentially usable as vaccines. It is especially concerned with the production of such preparations from bacteria of the genus *Bordetella*, particularly the species *Bordetella pertussis.*

It is known that the outer layer (commonly termed the envelope) of certain bacterial cells, including the species *Bordetella pertussis,* have antigenic properties and are protective when used as vaccines. However, many techniques of cell envelope isolation and fractionation are non-specific, destroy the protective activity or produce low yields of active material. For example, strong anionic surfactants, such as the alkali metal salts of *strong* acid esters (sodium dodecyl sulphate), are found to extract high yields of a material that is low in antigenic material (the low activity presumably being due to the denaturation of the protective components in the extract by the surfactant).

The unit activity of the extract may be increased by replacing a strong by a weak anionic surfactant, such as the alkali metal salt of a carboxylic acid. However, although extraction by such a carboxylic acid derivative, for example the sodium deoxycholate of UK Patent No 1,108,906 does lead to a higher unit activity than that achieved by sodium dodecyl sulphate (SDS), the extract still contains a number of unwanted non-immunogenic cell components.

The extent to which the protective components are denatured may be decreased still further by using a non-ionic surfactant, such as Triton X—100 (Trade Mark), for extraction. However, although the activity of the extract in this case is high the amount of material obtained is very low.

The choice of surfactant is therefore one of compromise, between high yield, low activity and low yield, high activity. None of the above surfactants have yet provided the best compromise in which the highest possible yield of material with the highest possible activity (lowest possible number of non-immunogenic cell components) is obtained.

According to the present invention, a process for producing an antigenically active cell envelope preparation from bacterial cells of the species *Bordetella pertussis* comprises disrupting the cells, separating the cell envelopes from the other cell components, extracting the separated envelopes with a solution of a surfactant and separating the cell envelope preparation from the resulting extract, characterised in that the surfactant is a zwitterionic surfactant. Preferably the extractant is an *N,N,N*-trialkyl zwitterionic compound, especially an *N,N,N*-trialkyl-α-amino acid, particularly an *N,N,N*-trialkyl glycine derivative, or an *N,N,N*-trialkyl amine oxide. More particularly, N-alkyl-N,N,-dimethylglycine derivatives or *N*-alkyl-*N,N*-dimethyl amine oxides, such as those sold under the trade names Empigen BB, OB or OY are preferred.

Zwitterionic surfactants are preferred to other classes of surfactants such as anionic and non-ionic surfactants, since they appear able to selectively extract high yields of antigenically-active material from the bacterial cells. For example, in a qualitative electrophoratic examination of the cell envelope preparations extracted from separated envelopes by sodium deoxycholate and Empigen BB (Trade Mark), an *N*-alkyl-*N,N*-dimethyl glycine, it was observed that the Empigen BB extract contained higher yields of antigenically active material, and gave fewer unidentified bands.

Zwitterionic surfactants, also known as amphoteric surfactants, are characterised in that each ion carries both a positive and negative charge. Thus zwitterionic surfactants resemble anionic surfactants such as SDS and sodium deoxycholate, in that each molecule contains equal and opposite electronic charges. However, unlike surfactants the charges in zwitterionic surfactants are not separable. On the other hand, zwitterionic surfactants also resemble non-ionic surfactants, in that the overall electronic charge of each molecule of both types of surfactant is zero. However, unlike non-ionic surfactants zwitterionic surfactants do contain electronic charges.

It appears, though the invention is not limited in any way by this explanation, that the beneficial properties of zwitterionic surfactants in the present invention derive from their dual character, of resembling both anionic and non-ionic surfactants. Thus they resemble anionic surfactants in their ability to extract sufficient protective material from the bacterial cells and non-ionic surfactants in their ability to retain antigenic activity.

The process of the invention may be applicable to any strain of the species *Bordetella pertussis*. Suitable strains include the isolate designated M2 available from Dr N W Preston, Departement of Bacteriology & Virology, University of Mnachester and the "Tohama" strain available from the Japanese Federation of Culture Collections of Microorganisms, and strain 134 available from Prof A Wardlaw. Department of Microbiology, Anderson College, 58 Dumbarton Road, Glasgow G11 6NU. However, the strain used does not appear to be critical and other strains may be used.

The cells may be produced by any conventional method, but are preferably cells which have been cultured in a liquid medium for not more than 36 hours since these generally produce cell envelope preparations with higher protective activity than those from longer term cultures. The cells are preferably disrupted by conventional mechanical techniques. Other techniques such as sonification or chemical disruption may be suitable in some cases, but are liable to cause undersirable changes in the cell envelope.

The extraction solution should normally contain between about 0.1% to 5%, expecially 0.5% to 5% (v/v) of the surfactant.

Preferably the surfactant concentration is 0.5% to 1.5% typically about 0.6% (v/v) of the surfactant. It should normally have a pH of less than 9 and preferably 7.5 to 8.5 and this is preferably achieved by use of a phosphate buffer. Other buffers may be suitable, but some, for example tris—HCl may reduce the activity of the product. The extraction period should be controlled to achieve maximum specificity of extraction and should, for example preferably be no more than 90 minutes at 37°C. The undissolved cell envelope material may be removed by centrifugation. The immunogenic component of the cell envelope extract may be precipitated from the extract for example by the addition of a lower alkyl (C1 to C5) alchohol, preferably ethanol, and may be dispersed in water and formulated into a vaccine in a conventional manner. Removal of any remaining surfactant from the precipitated material or original extract may be achieved by pressure dialysis against water or other conventional techniques.

The antigenically-active cell envelope preparation produced by extraction of cell walls with a zwitterionic surfactant in accordance with the present invention is a very much purer antigen preparation than either the original cells or the crude cell walls and hence may be expected to yield a vaccine less liable to produce side effects. However the preparation is still a mixture of proteins and hence may be susceptible to further separation to yield one or more antigen preparations. Hence the preparation prepared by the process of the present invention may be useful either as a vaccine in its own right or as an intermediate in the production of a vaccine. When used as a vaccine in its own right the dosage rate should typically be within the range of 2.5 to 250 mg per kg body weight. The vaccine may be used alone or with adjuvants and by single or multiple innoculation in accordance with conventional practices.

Specific embodiments of the process of the invention will now be described by way of example.

### Example 1

Cells of *Bordetalla pertussis* strain M2 were grown for 24 hours at 37°C in an aerated liquid medium as described by Cohen & Wheeler, American Journal of Public Health, Vol 36 (1946) pp 371—376.

The cells were disrupted by disintegration in a MSK Braun homogeniser for 4 minutes at 5°C. The cell envelopes were separated by differential centrifugation employing 2 washes of the envelopes in 0.9% (w/v) sodium chloride and 2 washes in distilled water. The produce was sterilised and freed from heat labile toxin by heating in an aqueous suspension to 56°C for 30 minutes.

The separated envelopes were shaken at a protein concentration of 5 mg/ml for 90 minutes at 37°C in an aqueous solution of 0.6% (v/v) of an *N*-alkyl *N,N* dimethyl glycine derivative (supplied by Marchon Division, Albright and Wilson Ltd under the Trade Mark Empigen BB) in a 0.05M sodium phosphate buffer (pH 8.0). The extract was separated by centrifugation and treated dropwise at 4°C with 40% of its own volume of ethanol. The precipitated materials was collected by centrifugation and dispersed in distilled water. Traces of surfactant were removed by further centrifugation followed by redispersal in distilled water. The traces of surfactant could also be removed by pressure dialysis against water.

The purified material was tested as a suspension in distilled water for protective activity by the vaccine mouse potency assay as recommended by Kendrick, Eldering, Dixon & Misner, American Journal of Public Health vol 37 (1947) pp 803—810 and found to be highly protective. The ethanol precipitate had a reduced lipopolysaccharide content compared with the soluble Empigen BB extract. The lipopolysaccharide content can be further reduced by column chromatography or other well known means.

### Example 2

The process of Example 1 was repeated on cells of *Bordetella pertussis* strain 134. The purified material was tested by the vaccine mouse potency assay and found to be highly protective.

### Example 3

The process of Example 1 was repeated on cells of *Bordetella pertussis* 'Tohama' strain to produce purified antigenic material.

### Claims

1. A process for producing an antigenically-active cell envelope preparation from bacterial cells of the species Bordetella pertussis comprising disrupting the cells, separating the cell envelopes from the other cell components, extracting the separated envelopes with a solution of a surfactant and separating the cell envelope preparation from the resulting extract, characterised in that the surfactant is a zwitterionic surfactant.

2. A process according to Claim 1 characterised in that the zwitterionic surfactant is an *N,N,N*-trialkyl glycine or an *N,N,N*-trialkyl amine oxide.

3. A process according to Claim 2 characterised in that the zwitterionic surfactant is an *N*-alkyl-*N,N*-dimethyl glycine or an *N*-alkyl-*N,N*-dimethyl amine oxide.

4. A process according to any one of Claims 1 to 3 characterised in that the extraction solution contains between 0.1% and 5% (v/v), and preferably between 0.5% and 5% (v/v), of the zwitterionic surfactant.

5. A process according to Claim 4 characterised in that the extraction solution contains between 0.5% and 1.5% (v/v) of the zwitterionic surfactant, and preferably about 0.6% (v/v) of the zwitterionic surfactant.

6. A process according to any one of Claims 1 to 5 characterised in that the extraction solution is buffered to a pH of less than 9.

7. A process according to claim 6 characterised in that the pH is between 7.5 and 8.5.

8. A process according to any one of Claims 1 to 7 characterised in that the extraction solution is buffered by a phosphate buffer.

9. A process according to any one of Claims 1 to 8 characterised in that the said cell envelope preparation is precipitated by the addition of a lower alkyl ($C_1$ to $C_5$) alcohol, preferably ethanol.

**Revendications**

1. Procédé pour la production d'une préparation de membranes cellulaires à propriétés antigéniques à partir de cellules bactériennes de l'espèce Bordetella pertussis comportant l'éclatement des cellules, la séparation des membranes des autres composants cellulaires, l'extraction des membranes séparées à l'aide d'une solution de surfactif et la séparation de la préparation des membranes cellulaires de l'extrait obtenu, caractérisé par le fait que le surfactif est un surfactif zwitterionique.

2. Procédé conforme à l'Article 1 caractérisé par le fait que le surfactif zwitterionique est un N,N,N-trialkyl glycocolle ou un N,N,N-trialkyl oxyde d'amine.

3. Procédé conforme à l'article 2 caractérisé par le fait que le surfactif zwitterionique est un N-alkyl-N,N-dimethyl glycocolle ou un N-alkyl-N,N-dimethyl oxyde d'amine.

4. Procédé conforme aux articles 1 à 3 caractérisé par le fait que la solution d'extraction contient entre 0.1% et 5% (v/v) et de préférence entre 0,5% et 5% (v/v) de surfactif zwitterionique.

5. Procédé conforme à l'Article 4 caractérisé par le fait que la solution d'extraction contient de 0,5% à 1,5% (v/v) de surfactif zwitterionique et de préférence 0,6% environ (v/v) de surfactif zwitterionique.

6. Procédé conforme aux Articles 1 à 5 caractérisé par le fait que la solution d'extraction est tamponnée à un pH de moins de 9.

7. Procédé conforme à l'Article 6 caractérisé par le fait que le pH est entre 7,5 et 8,5.

8. Procédé conforme aux articles 1 à 7 caractérisé par le fait que la solution d'extraction est tamponnée par un tampon de phosphate.

9. Procédé conforme aux articles 1 à 8 caractérisé par le fait que ladite préparation de membranes cellulaires est précipitée par l'addition d'un alcool alkyl inférieur ($C_1$ à $C_5$), de préférence de l'éthanol.

**Patentansprüche**

1. Ein Verfahren zur Erzeugung eines antigenetisch aktiven Zellwandpräparates aus Bakterienzellen der Gattung Bordetella pertussis durch Zerreißen der Zellen, Trennung der Zellwände von den übrigen Bestandteilen der Zellen, Auszug der getrennten Zellwände mit der Lösung eines oberflächenaktiven Mittels und Trennung der Zellwände vom resultierende Extrakt, dadurch charakterisiert, daß es sich bei dem oberflächenaktiven Mittel um eine zwitteronische oberflächenaktive Substanz handelt.

2. Ein Verfahren gemäß Patentanspruch 1, dadurch charakterisiert, daß die oberflächenaktive zwitteronische Substanz ein *N,N,N*-Trialkylglyzin oder ein *N,N,N*-Trialkylaminoxyd ist.

3. Ein Verfahren gemäß Patentanspruch 2, dadurch charakterisiert, daß die oberflächenaktive zwitteronische Substanz ein *N*-Alkyl-*N,N*-dimethyl-glyzin oder ein *N*-Alkyl-*N,N*-dimethylaminoxyd ist.

4. Ein Verfahren gemäß dem Patentansprüchen 1 bis 3, dadurch charakterisiert, daß die Extraktionslösung zwischen 0,1% und 5% (v/v) und vorzugsweise zwischen 0,5 und 5% der oberflachenaktiven zwitteronischen Substanz enthält.

5. Ein Verfahren gemäß Patentanspruch 4, dadurch charakterisiert, daß die Extraktionslösung zwischen 0,5% und 1,5% (v/v) und vorzugsweise etwa 0,6% (v/v) der oberflächenaktiven zwitteronischen Substanz enthält.

6. Ein Verfahren gemäß den Patentansprüchen 1 bis 5, dadurch charakterisiert, daß die Extraktionslösung auf einen pH-Wert unte 9 gepuffert ist.

7. Ein Verfahren gemäß Patentanspruch 6, dadurch charakterisiert, daß der pH-Wert zwischen 7,5 und 8,5 liegt.

8. Ein Verfahren gemäß den Patentansprüchen 1 bis 7, dadurch charakterisiert, daß die Extraktionslösung mit einem Phosphatpuffer gepuffert ist.

9. Ein Verfahren gemäß dem Patentansprüchen 1 bis 8, dadurch charakterisiert, daß besagtes Zellwandpräparat durch Zusetzen eines Alkohols mit niedrigwertigem Alkylrest ($C_1$ bis $C_5$), vorzugsweise Athylalkohol, ausgefällt wird.